# EUROPEAN PATENT APPLICATION

(11) **EP 3 785 752 A1**
(43) Date of publication of application: **03.03.2021**
(21) Application number: 19194448.7
(22) Date of filing: 29.08.2019
(51) Int. Cl.: A61M 5/32, A01G 13/00

(54) **DEVICE FOR INJECTING A LIQUID DOSE INTO A BANANA STEM**

(71) Applicant: Yara International ASA, 0277 Oslo (NO)
(72) Inventor: AGUILAR, Rolando Ureña, 281-6150 Santa Ana (CR)
(74) Representative: De Clercq & Partners

(57) **Abstract**

A device is disclosed for injecting a metered dose of a liquid into the soft tissue of a banana stem, comprising : (i) a liquid dose provision means configured to provide a metered dose of liquid to an adapter element upon actuation ; (ii) an adapter element adapted to be fitted onto the liquid dose provision means and having two adapter couplers, spaced apart from each other, and able to receive two conduit couplers of longitudinally extending needles, and equipped with means to provide the metered dose of liquid to said adapter couplers; (iii) two longitudinally extended needles for injecting liquid into the tissue of a banana stem, each of said needles comprising a tubular conduit having an outer surface, an inner conduit and a front and rear end portions, said front end portion formed into a tapered end, said outer surface comprising a longitudinal slit near said front end portion and parallel to the longitudinal axis of the tubular conduit and into which the inner conduit opens as an ejector hole such that liquid transported within said inner conduit may flow through said ejector and be deposited outside of said tubular conduit; and a conduit coupler attached to said rear end portion of said tubular conduit for connection to the adapter coupler such that liquid may flow through said conduit coupler, into said adapter coupler and into said tubular conduits, wherein the needles, when attached to the adapter element, are parallel to and spaced apart from each other. Also, a method is disclosed for use of the device according to the invention, in particular for injection of a liquid fertilizer into a banana tree pseudostem.

## Description

### Field of the Invention

This invention relates to banana stem injection systems and, more particularly, to a device for injecting liquids directly into the pseudostem of a banana plant.

### Background

A banana is an edible fruit produced by several kinds of large herbaceous flowering plants in the genus *Musa.* In some countries, bananas used for cooking may be called "plantains", distinguishing them from dessert bananas. The fruit is variable in size, colour, and firmness, but is usually elongated and curved, with soft flesh rich in starch covered with a rind, which may be green, yellow, red, purple, or brown when ripe. The fruits grow in clusters, also called bunches, hanging from the top of the plant. Almost all modern edible seedless bananas come from two wild species - *Musa acuminata* and *Musa balbisiana* and a hybrid - *Musa* × *paradisiaca.* The term "banana" is also used as the common name for the plants that produce the fruit. This can extend to other members of the genus *Musa,* such as the scarlet banana (*Musa coccinea*), the pink banana (*Musa velutina*), and the Fe'i bananas. It can also refer to members of the genus *Ensete,* such as the snow banana (*Ensete glaucum*) and the economically important false banana (*Ensete ventricosum*). Both genera belong to the banana family, *Musaceae.* In this description, when reference is made to a banana plant, it is meant to comprise all species of the banana family, *Musaceae.*

A mature banana plant consists of a pseudostem, which is not a tree, but is formed from tightly packed leaves, which supports the plant. Each pseudostem normally produces a single inflorescence, also known as the "banana heart". The banana fruits develop from the banana heart, in a large hanging cluster, made up of tiers (called "hands"), with up to 20 fruit per tier. The hanging cluster is known as a bunch, comprising 3 to 20 tiers, and can weigh 30 to 50 kilograms. Individual banana fruits (commonly known as a banana or "finger") average 125 grams, of which approximately 75% is water and 25% dry matter. After fruiting, the pseudostem dies, but offshoots (also called suckling) will normally have developed from the base, so that the plant as a whole is perennial.

In contrast to tree injection, which is the two-step operation of making a cut or incision in a tree with a first device and then placing a dose of chemical into the cut or incision using a second device, a banana stem injection is a one-step operation, wherein a spear or needle is inserted into the soft tissue of a banana pseudostem, in particular into the xylem vascular tissue, and an amount of a liquid is injected through said spear or needle.

Banana plant injection, also known as trunk or pseudostem injection, is a method described in the literature for the precise application of a dosage of e.g. pesticides, plant resistance activators, and fertilizers into the xylem vascular tissue of a banana plant with the purpose of protecting the plant from pests or for correction of nutrient deficiencies. This method largely relies on harnessing the plant's vascular system to translocate and distribute the active compounds into the pseudostem, canopy and roots where protection or nutrition is needed. Banana plant injection is currently only practiced for the application of Vydate® nematicide, and its nutritional use has not been documented, in particular for fertilizing banana plants.

Various aspects are associated with this injection method: since the banana stem consists of a spongy, soft material, it is imperative that the injection is not invasive and does not damage the stem tissue: it is very easy to manually drive a spear, stick, pole or needle into a banana (pseudo)stem. It is far more difficult to do this in a non-invasive way, leaving only a small wound. Wounds should be avoided as much as possible as they are entry points for insects and diseases. Furthermore, it is imperative that the injection is done in the outer tissue, and not in the core of the banana stem. As this kind of work is done in a routine way by workers in the field, the injection method needs to be simple and fail-safe: it should be impossible to inject into the core of the banana stem. Furthermore, the injection dose needs to be set precisely to dose just the right amount of fertilizer: too much may damage the plant and be an economical loss, a too low amount may be not effective; hence the device should be able to deliver a metered dosage amount.

### Background prior art

CN 105104031 (Hekou Yunshan Agic Sci & Technology, 2017) discloses a method and apparatus for administering a pesticide into banana (pseudo)stems using a backpack type dispensing device comprising a container with liquid inlet and outlet, straps allowing the container to be carried on the back of the user; a hose; a gun assembly and an injection device (syringe). A user carrying the device first inserts the injection needle (syringe) in the banana pseudostem and then activates the gun assembly to inject liquid in the banana pseudostem, typically 20 ml. This document is generally silent on administering fertilizers using the disclosed device.

CN107182582 discloses an injection system to apply fertilizer and pesticide into a banana pseudostem, comprising an injection gun and a needle, connected via a hose to a jerrycan-type container.

The open literature mentions the use of a calibrated Philips Drencher to inject Vydate L "Oxamyl" against banana nematodes (http://banana-networks.org/Bapnet/files-/2013/02/Truegellmann.pdf). The Philips Drencher is actually designed for oral application to sheep and cattle or for multiple dosing and has one needle.

The Banana Backpack Spear Injector (Sidewinder Pty Ltd, Queensland / http://treeinjectors.com/blog/banana-backpack-spear-injector) is an arm-operated banana-injector to inject kerosene, roundup and most insecticides. It comprises an arm-operated liquid holder to provide an amount of liquid and an aluminium hollow spear, e.g. with a standard length of 1 meter, carrying a single needle, to inject the liquid into the plant.

In general, an injection system is not widely used in banana plantations, except to apply the Vydate nematicide.

The inventors have now found a solution to the above-mentioned issues.

### Summary of the Invention

It is therefore an object of the present invention to provide an improved banana stem injection device.

Yet another object of the present invention is to provide a banana stem injection device which is relatively light in weight and easily portable and allows easy operation by the operator thereof.

A further object of the present invention is to provide a banana stem injection device which uses two needles to allow an efficient, rapid, error-free banana stem injection in one step, and divide the dose into two halves, with less internal damage of plant tissue.

Still another object of the present invention is to provide a banana stem injection device having dosage adjustment means to vary the amount of liquid introduced into a banana stem.

Another object of the present invention is to provide an improved banana stem injection method, based on the abovementioned one-step operation principle, which will cause relatively little damage to a banana stem.

Finally, an object of the present invention is to provide a device and method for banana stem injection, wherein the device is relatively simple to manufacture and safe and efficient in use, and wherein the method which is both time efficient and cost effective for the farmer.

According to one aspect, the invention relates to a device 1 for injecting a metered dose of a liquid into the soft tissue of a banana stem, comprising:
(i) a liquid dose provision means 2 configured to provide a metered dose of liquid to an adapter element 3 upon actuation;
(ii) an adapter element **3** adapted to be fitted onto the liquid dose provision means **2** and having two adapter couplers **4a, 4b,** spaced apart from each other, and able to receive two conduit couplers **5a, 5b** of longitudinally extending needles **6a, 6b,** and equipped with means to provide the metered dose of liquid to said adapter couplers **4a, 4b**;
(iii) two longitudinally extended needles **6a, 6b** for injecting liquid into the tissue of a banana stem, each of said needles comprising a tubular conduit **7a, 7b** having an outer surface **8a, 8b,** an inner conduit **9a, 9b** and a front **10a, 10b** and rear **11a, 11b** end portions, said front end portion **10a, 10b** formed into a tapered end, said outer surface **8a, 8b** comprising a longitudinal slit **12a, 12b** near said front **10a, 10b** end portion and parallel to the longitudinal axis of the tubular conduit **7a, 7b** and into which the inner conduit **9a, 9b** opens as an ejector hole **13a, 13b** such that liquid transported within said inner conduit **9a, 9b** may flow through said ejector hole **13a, 13b** and be deposited outside of said tubular conduit **7a, 7b** ; and a conduit coupler **5a, 5b** attached to said rear **11a, 11b** end portion of said tubular conduit **7a, 7b** for connection to the adapter coupler **4a, 4b** such that liquid may flow through said conduit coupler **4a, 4b,** into said adapter coupler **5a, 5b,** and into said tubular conduit **7a, 7b,** wherein the needles **6a, 6b,** when attached to the adapter element **3,** are parallel to and spaced apart from each other.

The device and various embodiments thereof are described in the appended depending claims. In this manner, upon penetration of the free end portion of the needle into a pseudostem to an extent beyond the ejector hole, liquid is injected through the liquid conduit and ejector hole into the banana pseudostem.

According to another aspect, the invention relates to a method for injecting a liquid into the soft tissue of a banana pseudostem having a diameter D, said method comprising the steps of:
(i) providing a device according to the invention, wherein the distance between the two vertical extended needles **6a, 6b** is less than the diameter D;
(ii) providing a container (**14**) for holding and dispensing the liquid;
(iii) providing a liquid in said container (**14**);
(iv) inserting said needles **6a, 6b** into the pseudostem of a banana plant such that said ejector holes **13a, 13b** are located interiorly of the outer surface of the pseudostem and such that the plane defined by the two needles **6a, 6b** is substantially perpendicular to the axis of the pseudostem of the banana plant;
(v) actuating liquid dose provision means **2,** thereby injecting a metered dose of liquid into the pseudostem of the banana plant; and
(vi) retracting said needles **6a, 6b** from the pseudostem of the banana plant.

In this manner relatively little damage to a banana stem is inflicted and a method which is both time efficient and cost effective for the farmer is provided.

The method and various embodiments thereof are described in the appended depending claims.

### Description of the drawings

Figure 1 shows a 3D drawing of an injector device according to the invention.
Figure 2 shows a 3D drawing of a needle.
Figure3 shows a 3D picture of a device according to the invention.
Figure 4 shows an assembly of injector device and container, carried by an operator.

### Detailed Description of the invention

Throughout the description and claims of this specification, the words "comprise" and variations of them mean "including but not limited to", and they are not intended to (and do not) exclude other moieties, additives, components, integers or steps. Throughout the description and claims of this specification, the singular encompasses the plural unless the context otherwise requires. In particular, where the indefinite article is used, the specification is to be understood as contemplating plurality as well as singularity, unless the context requires otherwise.

Features, integers, characteristics, compounds, chemical moieties or groups described in conjunction with a particular aspect, embodiment or example of the disclosure are to be understood to be applicable to any other aspect, embodiment or example described herein unless incompatible therewith. All of the features disclosed in this specification (including any accompanying claims, abstract and drawings), and/or all of the steps of any method or process so disclosed, may be combined in any combination, except combinations where at least some of such features and/or steps are mutually exclusive. The disclosure is not restricted to the details of any foregoing embodiments. The disclosure extends to any novel one, or any novel combination, of the features disclosed in this specification (including any accompanying claims, abstract and drawings), or to any novel one, or any novel combination, of the steps of any method or process so disclosed.

The enumeration of numeric values by means of ranges of numbers comprises all values and fractions in these ranges, as well as the cited end points. The term "ranges from ... to " as used when referring to a range for a measurable value, such as a parameter, an amount, a time period, and the like, is intended to include the limits associated to the range that is disclosed.

The term "approximately" and "about" as used when referring to a measurable value, such as a parameter, an amount, a time period, and the like, is intended to represent the measurement error associated with the technique to measure such parameter, amount, time period, and the like, and is considered to be 5% or less, depending on the measurement technique used. It should be understood that the value to which the term "approximately "and "about" refers per se has also been disclosed.

### Device

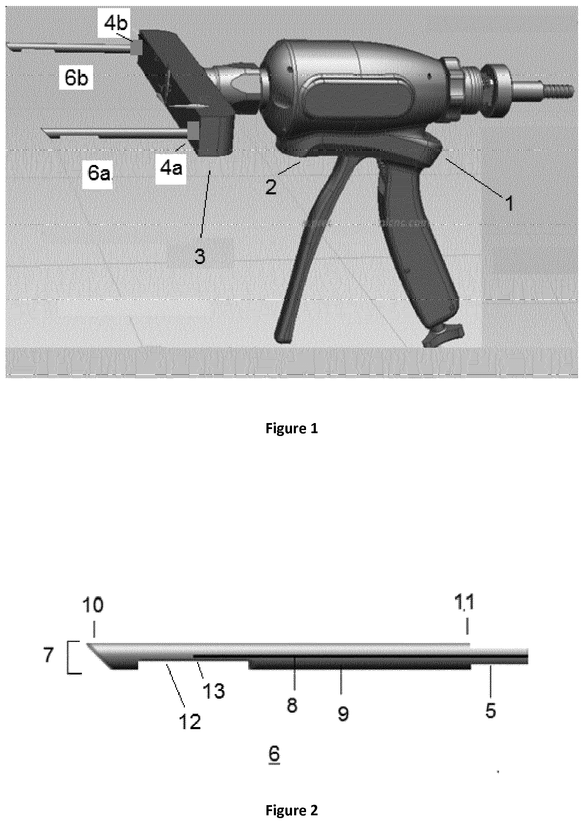

The device **1** is shown in its preferred embodiments in Figures 1 to 4.

According to one aspect, the invention relates to a device **1** for injecting a metered dose of a liquid into the soft tissue of a banana stem. The device comprises at least three main portions:
Firstly, the device **1** comprises a liquid dose provision means **2** configured to provide a metered dose of liquid to an adapter element **3** upon actuation. This can be any means, which is suitable to be used in the field, which is portable and can be operated by one person, and which is able to push a metered dose through a narrow conduit. Examples are the gun-type assembly Philips Drencher, and the Sidewinder Banana Backpack spear Injector (http://treeiniectors.com/blog/banana-backpack-spear-iniector/), which is equipped with a lever to pump liquid, and is operated by arm.

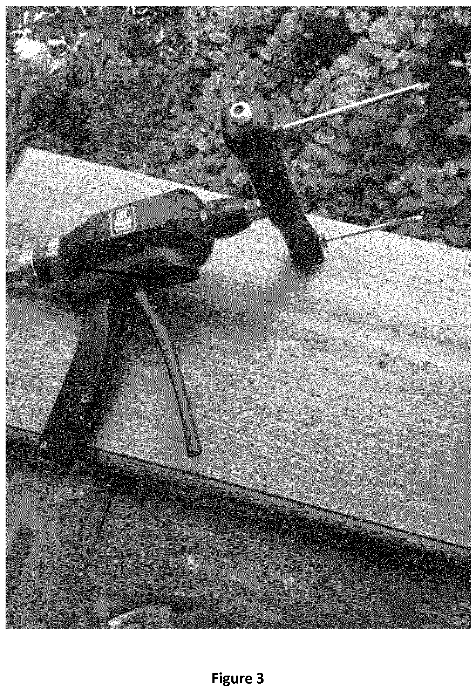

Most preferably, the liquid dose provision means are a hand-operatable gun assembly as illustrated in Figure 3.

The hand-operatable gun comprises an assembly of valves connected to a liquid chamber, being connected to a trigger, that open and/or close to load the chamber and/or expel the liquid out of the gun into the pseudostem of a banana plant.

When the trigger is operated, the liquid is injected, a valve is closed, and a vacuum is generated that forces liquid from the tank by suction into the liquid chamber to recharge the liquid chamber with the liquid at the selected dose.

The gun is connected to a container **14,** designed to hold and dispense liquid, such as a fertilizer, an insecticide, a pesticide, an herbicide, a fungicide or a nematicide liquid, wherein the liquid inside is held at atmospheric pressure, with connection means such as a plastic hose, constituting a fully closed system that is ready to load or inject a liquid dose into the liquid chamber.

The metered dose can be any amount that is useful for dosing to a banana plant. For example, in a copending application has been described the application of a novel fertilizer composition comprising 1 to 3 weight% of P (as P₂O₅), and 10 to 15 weight% of K (as K₂O), and having a pH of about 7, directly, i.e. without first making an incision, injected into the banana pseudostem for increasing the weight of the fruit of a banana plant, as a dose of 10 to 50 ml, preferable 25 ml.

The major difference with the prior art is that the invention is focussed on a double needle system to split the metered dose into two doses. To this end, the device **1** comprises an adapter element **3,** adapted to be fitted onto the liquid dose provision means **2** and having two adapter couplers **4a, 4b,** spaced apart from each other, and able to receive two conduit couplers **5a, 5b** of longitudinally extending needles **6a, 6b,** and equipped with means to provide the metered dose of liquid to said adapter couplers **4a, 4b**. The function of the adapter element **3** is to split the metered dose into two doses and guide the doses to the two adapter couplers **4a, 4b** which are designed to receive two conduit couplers **5a, 5b** of longitudinally extending needles **6a, 6b**.

According to one embodiment, the two adapter couplers **4a, 4b** are adjustably spaced apart from each other. In this way, the distance between the two needles **6a, 6b** can be adjusted to the diameter of the banana pseudostem to be injected. However, including such adjustment system into the injection device may raise manufacturing costs of the injection device. Any mechanical system to be used on banana farms by field workers must be very simple to operate, strong and easy to repair because many field workers may have little schooling, and working conditions in tropical climates involve the use of tools that are as simple as possible.

Therefor, according to another embodiment, the two adapter couplers **4a, 4b** are fixedly spaced apart from each other. For example, for the injection of a fertilizer composition, the size of the pseudostem is such that the distance between the two needles **6a, 6b** is about 15 cm. This distance was optimized and determined based on the average diameter of the banana stalks.

According to one embodiment, the doses guided to the two adapter couplers **4a**, **4b** can have a volume ratio of 1:1, injecting the same amount of liquid on the two different locations in the banana stem, but according to another embodiment, the split ratio may be different, such as 1:2. In this case, the adapter element **3** is provided with means to split the dose in two with a certain ratio, such as a valve arrangement. The split ratio could also be set manually, e.g. by providing a knob on the adapter element **3.**

According to an embodiment, the adapter element **3** is removably fitted onto the liquid dose provision means **2.** This allows for an easy exchange or removal of the adapter element **3,** e.g. in case of replacement or cleaning thereof.

Thirdly, as illustrated in Figure 2, the device **1** comprises two longitudinally extended needles 6 (**6a, 6b)** for injecting liquid into the tissue of a banana stem, each of said needles comprising a tubular conduit **7a, 7b** having an outer surface **8a, 8b,** an inner conduit **9a, 9b** and a front **10a, 10b** and rear **11a, 11b** end portions, said front end portion **10a, 10b** formed into a tapered end, said outer surface **8a, 8b** comprising a longitudinal slit **12a, 12b** near said front **10a, 10b** end portion and parallel to the longitudinal axis of the tubular conduit **7a, 7b** and into which the inner conduit **9a, 9b** opens as an ejector hole **13a, 13b** such that liquid transported within said inner conduit **9a, 9b** may flow through said ejector hole **12a, 12b** and be deposited outside of said tubular conduit **7a, 7b** ; and a conduit coupler **5a, 5b** attached to said rear **11a, 11b** end portion of said tubular conduit **7a, 7b** for connection to the adapter coupler **4a, 4b** such that liquid may flow through said conduit coupler **4a, 4b** into said adapter coupler **5a, 5b,** and into said tubular conduits **7a, 7b,** wherein the needles **6a, 6b,** when attached to the adapter element **3,** are parallel to and spaced apart from each other.

The needles **6a, 6b** are designed to convey the metered dose with a minimum of damage to the plant tissue. Normally, an ejector hole is located at the tip of the needle. To prevent clogging of the ejector hole when the needle is inserted multiple times in soft tissue, the ejector hole **13a, 13b** has been arranged in a longitudinal slit **12a, 12b,** opening to the side of the needle. The dose is therefore not ejected at a depth equal to the length of the needle, but at a depth which is less than the length of the needle.

According to one embodiment, one or both needles **6a, 6b** may be provided with a depth indicator, such as a scale, to measure the depth the needles are inserted into the pseudostem. Alternatively, one or both needles may be provided with an indicator of maximal depth, such as a mark on the needle, or a stopper, to indicate a certain depth the needles should or can be inserted into the pseudostem. Alternatively, the adapter element **3** may be fitted with a depth indicator, like a conventional drill.

According to one embodiment, the combination of conduit coupler **4a, 4b** and adapter coupler **4a, 4b** provides a separable arrangement wherein the needle is removable and allows an easy exchange of the needle, e.g. in case of replacement or cleaning thereof.

According to another embodiment, the combination of conduit coupler **4a, 4b** and adapter coupler **4a, 4b** provides an inseparable arrangement where the needle is not removable.

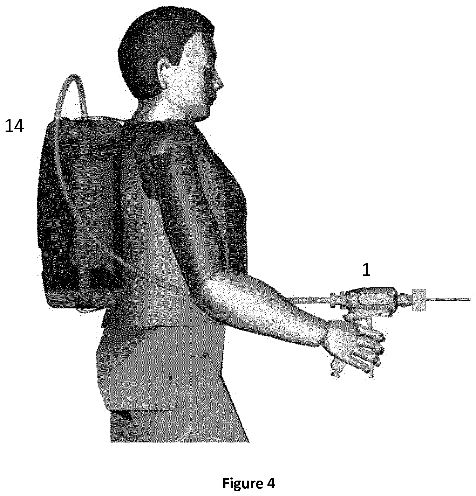

According to one embodiment, the device **1** further comprises a container **14** for holding and dispensing the liquid, in communication with the liquid dose provision means **3** for providing the liquid to the liquid dose provision means **3.** The container **14** is designed to hold and dispense a liquid, such as a fertilizer, an insecticide, a pesticide, an herbicide, a fungicide, a nematicide or any other liquid. Preferably, the container **14** is designed as a backpack to be carried on the back of the operator (Figure 4). It can be provided with straps to strap the container **14** onto the back of the operator, or it can be designed as a unit to be inserted into a bay which is strapped onto the back of the operator. The latter design allows a quick and easy removal and exchange of a container **14** without the need for unstrapping. Both type of containers can be designed as refillable containers.

According to one embodiment, the device **1** according to the invention may further comprise a valve switch to close off the feed of liquid to the inner conduits **9a, 9b,** that is activated when the device **1** is held in a position such that a plane defined by the two needles **6a, 6b** is in a substantial vertical alignment with the vertical axis of the pseudostem of the banana plant. This has the advantage that it is virtually impossible to inject a dose in the central part of the banana pseudostem, and the device **1** needs to be operated with the two needles in a plane which is substantially horizontal with reference to the vertical axis of the pseudostem of the banana plant.

### Method

According to one aspect of the invention, there is provided a method for injecting a liquid into the soft tissue of a banana pseudostem having a diameter D using the device according to the invention.

The method comprises 6 steps. In a first step, a device according to the invention is provided, wherein the distance between the two vertical extended needles **6a, 6b** is less than the diameter D. It is advantageous that the distance between the two vertical extended needles **6a, 6b** can be adjusted. According to one embodiment, said distance is about 15 cm.

In a second step, a container **14** is provided for holding and dispensing the liquid. We refer to the above paragraphs for the details on the container **14.**

In a third step, a liquid is provided in said container **14.** We refer to the above paragraphs for the details on the liquid.

In a fourth step, the operator holding the device **1** inserts said needles **6a, 6b** into the pseudostem of a banana plant such that said ejector holes **13a, 13b** are located interiorly of the outer surface of the pseudostem and such that the plane defined by the two needles **6a, 6b** is substantially perpendicular to the axis of the pseudostem of the banana plant. In view of the soft tissue, little force is necessary to penetrate the outer surface of the pseudostem. On the contrary, attention needs to be given not to insert the needles too far into the tissue. A depth indicator or stopper could be very useful here.

In a fifth step, said liquid dose provision means **2** are actuated, thereby injecting a metered dose of liquid into the pseudostem of the banana plant.

In a sixth step, said needles **6a, 6b** are retracted from the pseudostem of the banana plant by retracting the device **1.**

According to one embodiment, the method is performed wherein the distance between the two vertical extended needles (6a, 6b) is more than the diameter D and wherein the plane defined by the two needles is tilted with respect to the axis of the pseudostem of the banana plant over an angle α, which angle is sufficient to inject liquid into the outer surface of the pseudostem but not inject the liquid into the centre of the pseudostem of the banana plant. This embodiment allows injection into pseudostems of which the diameter is smaller than the distance between the two needles **6a, 6b**. The device can be tilted, and the needles are inserted such that one needle penetrates the pseudostem at one side at a higher point than the other needle at the other side. This allows the use of a device **1** wherein the two adapter couplers **4a, 4b** are fixedly spaced apart from each other, or the two adapter couplers **4a, 4b** are adjustably spaced apart from each other but it requires too much effort to adjust the distance with each plant.

This method will cause relatively little damage to a banana stem and is both time efficient and cost effective for the farmer. The device and the method according to the invention are very suited for injection of a liquid into a banana tree pseudostem, in particular a liquid fertilizer. In particular, the liquid fertilizer is an aqueous fertilizer composition comprising 1 to 3 weight% of P (as P₂O₅), and 10 to 15 weight% of K (as K₂O) and having a pH of about 7.

Furthermore, it is to be understood that numerous modifications and adjustments to both the device and the method of the present invention may be contemplated and performed. For example, a completely different liquid dose provision means than the one described above may be used in conjunction with the two-needle arrangement according to the invention. Alternatively, the needle itself may include a plurality of ejection holes. Therefore, the above description is not intended in any way to limit the scope of the present invention, the scope of which shall follow in the claims as set forth below.

## Claims

1. Device (1) for injecting a metered dose of a liquid into the soft tissue of a banana stem, comprising :
(i) a liquid dose provision means (2) configured to provide a metered dose of liquid to an adapter element (3) upon actuation ;
(ii) an adapter element (3) adapted to be fitted onto the liquid dose provision means (2) and having two adapter couplers (4a, 4b), spaced apart from each other, and able to receive two conduit couplers (5a, 5b) of longitudinally extending needles (6a, 6b), and equipped with means to provide the metered dose of liquid to said adapter couplers (4a, 4b);
(iii) two longitudinally extended needles (6a, 6b) for injecting liquid into the tissue of a banana stem, each of said needles comprising a tubular conduit (7a, 7b) having an outer surface (8a, 8b), an inner conduit (9a, 9b) and a front (10a, 10b) and rear (11a, 11b) end portions, said front end portion (10a, 10b) formed into a tapered end, said outer surface (8a, 8b) comprising a longitudinal slit (12a, 12b) near said front (10a, 10b) end portion and parallel to the longitudinal axis of the tubular conduit (7a, 7b) and into which the inner conduit (9a, 9b) opens as an ejector hole (13a, 13b) such that liquid transported within said inner conduit (9a, 9b) may flow through said ejector hole (13a, 13b) and be deposited outside of said tubular conduit (7a, 7b); and a conduit coupler (5a, 5b) attached to said rear (11a, 11b) end portion of said tubular conduit (7a, 7b) for connection to the adapter coupler (4a, 4b) such that liquid may flow through said conduit coupler (4a, 4b), into said adapter coupler (5a, 5b) and into said tubular conduits (7a, 7b), wherein the needles (6a, 6b), when attached to the adapter element (3), are parallel to and spaced apart from each other.

2. Device (1) according to claim 1, wherein the liquid dose provision means is a hand-operatable gun assembly.

3. Device (1) according to any one of claims 1 to 2, wherein the two adapter couplers (4a, 4b) are adjustably spaced apart from each other.

4. Device (1) according to claim 3, wherein the two adapter couplers are fixedly spaced apart from each other, preferably at a distance of about 15 cm.

5. Device (1) according to anyone of claims 1 to 4, wherein the adapter element (3) is provided with means to split the dose in two with a certain ratio.

6. Device (1) according to any one of claims 1 to 5, further comprising
(iii) a container (14) for holding and dispensing the liquid, in communication with the liquid dose provision means (3) for providing the liquid to the liquid dose provision means (3).

7. Device (1) according to claim 6, wherein the container (14) is a backpack-type container that can be strapped onto the back of a person.

8. Device (1) according to claim 6, wherein the container (14) is a unit-type container that can be inserted into a bay, strapped onto the back of a person.

9. Device (1) according to any one of claims 6 or 8, wherein the container (14) is refillable and/or wherein the liquid inside the container is held at atmospheric pressure.

10. Device (1) according to any one of claims 1 to 9, further comprising a valve switch to close off the feed of liquid to the inner conduits (9a, 9b), that is activated when the device (1) is held in a position such that a plane defined by the two needles (6a, 6b) is in a substantial vertical alignment with the vertical axis of the pseudostem of the banana plant.

11. A method for injecting a liquid into the soft tissue of a banana pseudostem having a diameter D, said method comprising the steps of :
(i) providing a device according to any one of claims 1 to 10, wherein the distance between the two vertical extended needles (6a, 6b) is less than the diameter D;
(ii) providing a container (14) for holding and dispensing the liquid;
(iii) providing a liquid in said container (14);
(iv) inserting said needles (6a, 6b) into the pseudostem of a banana plant such that said ejector holes (13a, 13b) are located interiorly of the outer surface of the pseudostem and such that the plane defined by the two needles (6a, 6b) is substantially perpendicular to the axis of the pseudostem of the banana plant;
(v) actuating said liquid dose provision means (2), thereby injecting a metered dose of liquid into the pseudostem of the banana plant; and
(vi) retracting said needles (6a, 6b) from the pseudostem of the banana plant.

12. The method according to claim 11, wherein the distance between the two vertical extended needles (6a, 6b) is more than the diameter D and wherein the plane defined by the two needles (6a, 6b) is tilted with respect to the axis of the pseudostem of the banana plant over an angle α, which angle is sufficient to inject liquid into the outer surface of the pseudostem but not inject the liquid into the centre of the pseudostem of the banana plant.

13. The method according to any one of 11 to 12, wherein the liquid is one of a fertilizer, an insecticide, a pesticide, an herbicide, a fungicide or a nematicide.

14. The method according to any one of claims 11 to 13, wherein the liquid is an aqueous fertilizer composition comprising 1 to 3 weight% of P (as P₂O₅), and 10 to 15 weight% of K (as K₂O) and having a pH of about 7.

15. Use of the device according to any one of claims 1 to 10 for injection of a liquid into a banana tree pseudostem, in particular a liquid fertilizer.
